# EUROPEAN PATENT APPLICATION

(11) **EP 1 550 470 A1**
(43) Date of publication of application: **06.07.2005**
(21) Application number: 03730734.5
(22) Date of filing: 30.05.2003
(51) Int. Cl.: A61L 27/00, A61K 35/32

(54) **METHOD OF REGENERATING TOOTH GERM AND REGENERATED TOOTH GERM**

(30) Priority: 31.05.2002 JP 2002158710
(71) Applicant: Hitachi Medical Corporation, Tokyo 101-0047 (JP); Ueda, Minoru, Nagoya-shi, Aichi 461-0011 (JP)
(72) Inventor: UEDA, Minoru, Nagoya-shi, Aichi 461-0011 (JP); HONDA, Masaki, Nisshin-shi, Aichi 470-0114 (JP); KAGAMI, Hideaki, Kita-ku, Nagoya-shi, Aichi 462-0831 (JP); SHIMIZU, Hiromichi 303, Dai-7 Takeishimansion, Katsushika-ku, Tokyo 125-0033 (JP)
(74) Representative: Strehl Schübel-Hopf & Partner
(86) International application number: PCT/JP2003/006843
(87) International publication number: WO 2003/101503

(57) **Abstract**

An object of the present invention is to provide a method for regenerating tooth germ, and more specifically, to provide a method for regenerating tooth germ that enables the treatment of patients who have lost teeth or have had teeth damaged by dental diseases such as pyorrhea alveolaris or dental caries. The present invention provides a method for regenerating tooth germ by culturing tooth germ cells while giving mechanical stimulus to the cells, and a regenerated tooth germ.

## Description

### TECHNICAL FIELD

The present invention relates to a method for regenerating tooth germ. More specifically, the present invention relates to a method for regenerating tooth germ by culturing tooth germ cells while giving mechanical stimulus to the cells. The present invention also relates to a method for treating patients with dental diseases using tooth germ regenerated by the above method.

### BACKGROUND ART

The modem society is an aging society, and it is predicted that elderly people over age 65 will make up approximately 20% of the total population in Japan in several years. A majority of such elderly people have lost a part or all of their teeth for various reasons, and many of them use artificial teeth (what are called "false teeth"). The conventional artificial tooth has been problematic, not only in that it requires to be put on and taken off and causes an uncomfortable feeling when it is attached, but also in that the use thereof imposes psychological pressure upon patients, giving an impression as a symbol of aging. Thus, it is recognized that patients are generally reluctant to use such an artificial tooth. In addition, it has been known that when full dentures are attached because all teeth were lost, chewing ability becomes approximately one-fifth of that of natural teeth. It is not negligible that eating, which may be a pleasure for many elderly people, often causes pain after the loss of teeth. Moreover, it has been clarified that the masticatory stimulus upon the brain has effects of preventing dementia, and thus, that a decrease in chewing ability promotes dementia.

Under these circumstances, a dental implant has been developed and applied to clinical sites in recent years. Application of such a dental implant has achieved the fixing of artificial teeth, allowed for easy maintenance, and improved chewing ability. However, it has not yet been satisfactory in terms of esthetics or comfortable fitting. Moreover, it cannot be said that implant dentures have widely been used, for the reasons that it requires surgery; that it requires a certain amount of bone, and thus, the use of the dental implant is restricted depending on the general status of a patient; and that it has a high cost, and further reliable medical institutions are also limited. Consequently, although there are many patients who use artificial teeth and are not satisfied with them, only a very limited number of patients use implant dentures.

Transplantation of teeth by allotransplantation has been reported. However, it is difficult to remove and maintain transplantable healthy teeth, and such type of tooth transplantation involves the risk of infectious diseases. Thus, allotransplantation has not yet become a common treatment. There are many patients who do not venture to try dental implants although they are not satisfied with artificial teeth, or who have difficulty in undergoing a treatment with implant dentures due to their individual conditions.

To date, regarding studies of dental regeneration, regeneration of periodontal tissues have become a focus of attention, and related studies have been undertaken mainly for regeneration of bones and periodontal membranes. As a result of these studies, the GTR method (Guided Tissue Regeneration method) has been developed. The GTR method involves preventing epidermic cells from entering the surface of a tooth root, using a membrane such as Millipore Filter (product name; Millipore Corp.), so as to form a space necessary for the growth of periodontal cells (Nyman et al., J. Clin. Periodontol., 9, 290 (1982)). The GTR method intends to regenerate alveolar bones and periodontal membranes around teeth affected by periodontal disease. This method yields good results in the case of mild periodontal diseases. Moreover, in recent years, a protein capable of regenerating a periodontal membrane has been developed and practically used. However, the GTR method cannot be applied when there is a high degree of absorption regarding alveolar bones, which causes the loss of teeth. Further, it cannot repair the collapse of teeth caused by dental caries.

In order to fundamentally solve the aforementioned problems, a method for regenerating tooth germ itself has been proposed and studied (C. S. Young et al J. Dent. Res. 81 (10), 695-2000, 2002; and Tissue Engineering, Vol. 7, Number 5, 624, October 2001). However, only small tissues have been formed by this method, and tissues that were large enough to be used in practical treatment have not been formed.

### DISCLOSURE OF THE INVENTION

It is an object of the present invention to solve the aforementioned problems of the prior art. In other words, it is an object of the present invention to provide a method for regenerating tooth germ, and more specifically, to provide a method for regenerating tooth germ that enables the treatment of patients who have lost teeth or have had teeth damaged by dental diseases such as pyorrhea alveolaris or dental caries. Moreover, it is another object of the present invention to provide a method for treating patients who have lost teeth or have had teeth damaged, using regenerated tooth germ.

As a result of intensive studies directed towards achieving the aforementioned objects, the present inventors have found that induction of differentiation of tooth germ cells can be promoted by culturing the tooth germ cells while giving mechanical stimulus to the cells, and further, that the survival rate of the cultured tooth germ cells can also be improved by giving such mechanical stimulus, thereby completing the present invention.

Thus, the present invention provides a method for regenerating tooth germ by culturing at least one type of tooth germ cells and any cells capable of differentiating into the tooth germ cells, while giving mechanical stimulus to the cells.

The above-mentioned at least one type of cells are preferably odontoblasts, ameloblasts, pulp or dental papilla cells, tooth sac cells, or precursor cells thereof.

Preferably, the above-mentioned at least one type of cells are obtained by fragmenting tissues collected from a living body, treating the fragmented tissues with enzyme, and separating and recovering them.

Preferably, the above-mentioned at least one type of cells are inoculated on a carrier, and the cells are cultured on the carrier, while giving mechanical stimulus thereto.

As such a carrier, there is preferably used a carrier, the material of which has affinity to a living body into which the cultured cells are transplanted and an ability to be absorbed into the living body, and which has a form of interest to be regenerated and also has a portion into which blood circulation is introduced.

The above-mentioned carrier preferably consists of at least one selected from polyglycolic acid (PGA), poly(DL-lactide-co-glycolide) (PLGA), poly(DL-lactide) (PLLA), caprolactone, collagen, or natural materials such as dentin.

The above-mentioned carrier preferably has at least one form selected from mesh, sponge, or gel.

Preferably, at least one mechanical stimulus selected from shake culture, ultrasonification, extension stimulus, or culture under pressure, is given to the above-mentioned at least one type of cells.

There is preferably provided a method for regenerating tooth germ, which is characterized in that the cells of the above-mentioned at least one type of cell, which have been cultured while giving the above mechanical stimulus thereto, are transplanted into the body of an animal, so as to allow tooth germ to regenerate in the body of the above animal.

The above animal is preferably a mammal.

Preferably, the above-mentioned at least one type of cells are transplanted into a site of the body of the above animal, which has a high blood flow.

There is preferably provided a method for regenerating tooth germ, which is characterized in that the above-mentioned at least one type of cells are inoculated on the above carrier, and that the cells are subjected to shake culture on the carrier at a frequency of approximately 50 times/minute, so as to promote the induction of differentiation and the growth.

There is preferably provided a method for regenerating tooth germ, which is characterized in that it comprises inoculating the above-mentioned at least one type of cells on the above carrier, subjecting the cells to shake culture on the carrier at a frequency of approximately 50 times/minute, so as to promote the induction of differentiation and the growth, transplanting the cells into the body of an animal, and excising the regenerated tooth germ after approximately 15 weeks.

The present invention also provides tooth germ regenerated by the above-mentioned method of the present invention.

The above regenerated tooth germ preferably comprises at least one selected from dentin, dental papilla, or enamel pulp.

The present invention further provides a treatment method, which is characterized in that tooth germ regenerated by any one of the above-mentioned methods is transplanted into the jaw of a patient who has lost his or her own tooth germ or has had his or her own tooth germ damaged, so as to provide the patient with the regenerated germ tooth.

### BRIEF DESCRIPTION OF THE DRAWINGS

Figure 1 is a view showing the state of cells at 15 weeks after transplantation. In Figure 1A, tooth-germ-like hard tissues are formed, and a calcified product that is considered to be dentin is observed. In addition, calcification is promoted by mechanical stimulus.
Figure 2 is a view showing the comparison made between the size of the formed tooth-germ-like tissues in the case of adding mechanical stimulus (+) and that in the case of not adding mechanical stimulus (-).
Figure 3 shows the results of RT-PCR.
Figure 4 shows a change over time in alkaline phosphatase activity in the presence or absence of mechanical stimulus.
Figure 5 shows the comparison of expression of amelogenin mRNA in the presence or absence of mechanical stimulus. ((+) represents the presence of mechanical stimulus and (-) represents the absence of mechanical stimulus.)

### BEST MODE FOR CARRYING OUT THE INVENTION

The embodiments of the present invention will be described in detail below.

The method of the present invention for regenerating tooth germ is characterized in that tooth germ cells are cultured while mechanical stimulus is given to the cells.

The type of the tooth germ cell used in the present invention is not particularly limited, as long as the cell constitutes tooth germ or can differentiate into a tooth germ cell. Examples of such cells may include odontoblasts, ameloblasts, pulp or dental papilla cells, tooth sac cells, or precursor cells thereof. These cells may be cultured as single cell consisting of one type of cell, or may also be cultured as a mixture of cells consisting of two or more types of cells.

Tooth germ cells can be collected from the lower jaw of a mammal (for example, a human, a swine, etc.). An impacted tooth is aseptically excised, and it is then conserved in a suitable preservation solution such as a Hanks balanced salt solution (HBSS). A calcified portion is removed from the tooth, and the residual tissues are fragmented using a knife. The fragmented tissues are then washed with an HBSS solution or the like. Subsequently, the tissues are preferably subjected to an enzyme treatment with collagenase and dispase. After completion of such an enzyme treatment, cells are recovered by pipetting and centrifugation.

The tooth germ regenerated by the method of the present invention is transplanted to a dental patient (that is, a patient who suffers from the loss of a tooth or a damaged tooth), and thus, it is used in the treatment of such a patient. In this case, from the viewpoint of biocompatibility associated with transplantation, tooth germ cells used in regeneration are preferably the patient's own tooth germ cells. The cells which constitute tooth germ or the cells which can differentiate into tooth germ can also be collected from wisdom teeth.

It has been known that a tooth is formed via 5 stages ranging from generation to maturation. The first stage is called the initiation stage, when epithelial tissues and mesenchymal tissues are induced to the basement membrane. The second stage is called the bud stage, when an enamel organ is generated. The third stage is called the cap stage, when dental papilla is generated and tooth germ is then generated. The fourth stage is called the bell stage, when both differentiation of the tooth germ into cells forming dental enamel and differentiation of the dental papilla into cells forming dentin and dental pulp are initiated. The fifth stage is called the maturation stage, when cells are differentiated into tissues constituting the tooth, such as dental enamel, dentin, and dental pulp. In the present invention, any cells in a preferred stage selected from the aforementioned stages can be collected and used. In a case where no tooth germ exists, dental pulp or periodontal membrane is excised from a tooth root, and cells can be then separated and collected therefrom. It is to be noted that excision of dental pulp from a tooth can be carried out by the method described in About I et al., Experimental Cell Research. 258. 33-41, 2000.

In the present invention, the term "regeneration of tooth germ" is used to mean regeneration of tooth germ obtained from the second stage onwards, from among the aforementioned 5 stages.

In the present invention, tooth germ cells are cultured, while mechanical stimulus is given to the cells. Mechanical stimulus can be given, for example, by subjecting the cells to shake culture, performing ultrasonification on the cells, giving extension stimulus to the cells, culturing the cells under pressure, etc. Preferably, the cells are subjected to shake culture. In such shake culture, a vessel containing cells and a medium is placed on a shaker, and it is then shaken at an appropriate rate (for example, approximately 10 to 100 times/minute), so as to culture the cells.

The cells can be cultured, using a common medium containing serum used in the culture of animal cells, under common conditions for culturing animal cells (for example, at a temperature between room temperature and 37°C, in a 5% CO₂ incubator).

When stimulus is given to tooth germ cells in the present invention, tooth germ cells may be cultured on a carrier, or may be cultured with no carriers. However, tooth germ cells are preferably cultured on a carrier. The use of a carrier is advantageous for forming tooth germ tissues from cells. It is preferable to use a carrier, which endures a period of time necessary for formation of tooth germ, and which is then rapidly absorbed into a body. That is to say, it is preferable to use a carrier, which has a suitable absorption speed and suitable properties in a living body such as the greater omentum attached to the stomach, or the jaw, and the material of which has high affinity to cells.

The material of such a carrier is not particularly limited, as long as it satisfies the aforementioned properties. Examples of such a material may include polyglycolic acid (PGA), poly(DL-lactide-co-glycolide) (PLGA), caprolactone, collagen. Alternatively, natural materials such as dentin can also be used.

PGA is commercially available from Albany International Research Co. and other companies. PLGA is commercially available from Sigma (cat. P1816). In the case of PGA, since this compound is rapidly absorbed, it is also possible to coat the surface thereof with poly(DL-lactide) (PLLA), so as to retard the absorption period. Moreover, when synthetic materials such as PGA, PLGA, or caprolactone are used, the surfaces of these compounds are coated with a collagen solution and then used, in order to enhance the adhesiveness of cells.

Examples of a possible form as a carrier may include a mesh form, a sponge form, and a gel form. In the case of a carrier having a gel form, cells come into contact with one another more easily than in the case of a carrier having a mesh or sponge form. Accordingly, among others, a carrier having a gel form is most useful for the culture of tooth germ cells.

There is preferably used a carrier that is processed into a form, which facilitates transplantation of cells. Such a carrier preferably has a platy, spherical, or hollow form, one end of which is open, having a portion into which blood circulation can easily be introduced from surrounding portions.

It is preferable to produce a carrier having a form that is suitable for purpose. Thus, a form of interest is produced from resin, and then, a mold is obtained using an impression material. Thereafter, the mold of resin is taken out, and a synthetic material constituting a carrier is poured therein, so as to replicate a form of interest.

In the method of the present invention, tooth germ cells may be cultured while mechanical stimulus is given thereto, and then the cultured cells may be transplanted into a transplanted animal, and tooth germ may be regenerated in the body of the transplanted animal. Alternatively, the above-mentioned cultured cells may be directly transplanted into the jaw of a patient. Preferably, a carrier used in the culture of tooth germ cells is transplanted into the body of the transplanted animal together with the tooth germ cells.

The type of a transplanted animal is not particularly limited, but it is preferably a mammal. Examples of a mammal used herein may include rodents such as a rat (e.g. a nude rat), rabbit, or mouse. As a site into which tooth germ cells are transplanted, a site to which factors necessary for formation of tooth germ can easily be supplied is preferable. More specifically, a site having a high blood flow, such as the greater omentum attached to the stomach in the abdominal cavity, is particularly preferable. By transplanting tooth germ cells into such a site, the growth of the tooth germ cells can be promoted, and formation of tooth germ can be accelerated.

Tooth germ regenerated by the above-mentioned method of the present invention for regenerating tooth germ (which may be either tooth germ tissues obtained by culturing tooth germ cells while giving mechanical stimulus thereto, or tooth germ tissues obtained by transplanting the above-mentioned tooth germ tissues into a transplanted animal and allowing them to further regenerate in the body of the transplanted animal) is transplanted into the jaw of a patient who has lost a tooth or has had a tooth damaged, so as to treat the dental patient. That is to say, a method for treating a dental patient using tooth germ obtained by the method of the present invention for regenerating tooth germ, is also included in the scope of the present invention. Even after tooth germ has been transplanted into the jaw of a dental patient, the tooth germ is allowed to continuously grow, so as to form a tooth. Otherwise, a tooth root may be formed from tooth germ outside the body of a patient. The tooth root may be transplanted into the jaw of the patient, and a tooth crown may be then formed by the conventional dental methods.

The present invention will be further described in detail by the following Examples. However, the Examples are not intended to limit the scope of the present invention.

### EXAMPLES

### Example 1: Regeneration of tooth germ using mechanical stimulus

The lower jaw was collected from a fresh swine of several weeks old. The lower jaw was conversed in a refrigerator at 4°C until it was used for experiments. During transportation, it was conversed on ice. An impacted tooth was aseptically excised, and it was then conserved in a Hanks balanced salt solution (HBSS). A calcified portion was removed from the tooth, and using a knife, the residual tissues were fragmented into fragments with a size of about 2 mm. The fragmented tissues were then washed with an HBSS solution approximately 5 times.

The washed tissues were subjected to an enzyme treatment for 30 minutes, using an enzyme solution obtained by dissolving collagenase (2 mg/ml) and dispase I1A in 35 ml of an HBSS solution.

Thereafter, pipetting was carried out using a 25 ml pipette. 25 ml of a supernatant was centrifuged (1,500 rpm, 10 minutes) to recover cells (1,500 rpm, 10 minutes) (referred to as recovery cell 1). The residual tissues contained in 10 ml of a collagenase solution were subjected to pipetting with a 10 ml pipette for 10 minutes (referred to as recovery cell 2). Recovery cell 1 was mixed with recovery cell 2, and the mixture was again centrifuged, so as to recover cells. The thus obtained cells were washed 3 times with a medium containing 10% serum, and then centrifuged, so as to recover the cells.

The isolated cells were adjusted to a concentration of 1 x 10⁶ cells/ml, and 3 x 10⁶ cells were inoculated on a single carrier (a collagen sponge carrier (a vacancy rate of 99%, Nipro Corp., Japan)). In an experiment group, the carrier on which the cells had been inoculated was shaken at 50 times/minute in a shaker for 12 hours before transplantation. In a control group, a static culture was carried out for 12 hours. As mediums for culturing the cells, a medium prepared by adding 10% fetal bovine serum to Dulbecco's Modified Eagle Medium, and a medium containing glutamic acid and antibiotics, were used. Culture of the cells was carried out under conditions of 37°C and 5% CO₂.

F344 nude rat was used as a transplanted animal. The abdominal cavity of the F344 nude rat was opened, and the cultured cells were transplanted into the greater omentum. Thereafter, the cultured cells were excised after transplantation for 15 weeks.

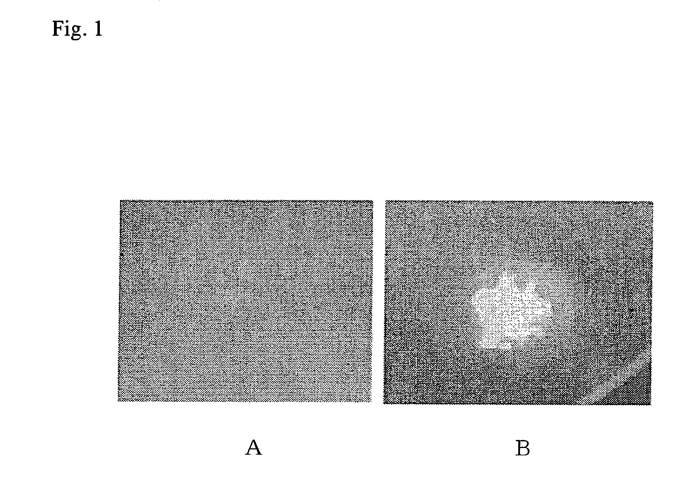

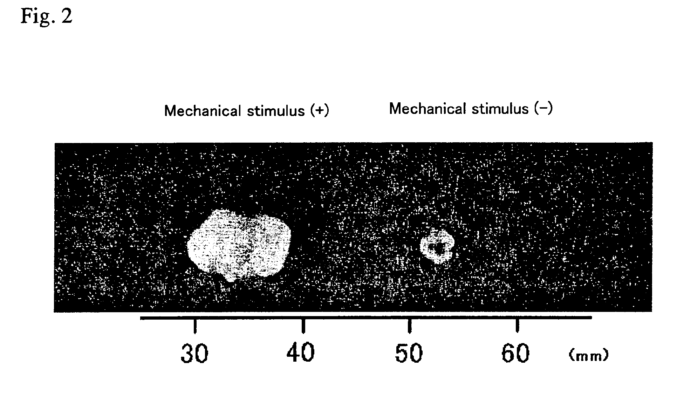

The transplanted cells formed tooth-germ-like hard tissues during 15 weeks, and a calcified product that was considered to be dentin was observed (Figure 1A). Moreover, calcification was promoted by mechanical stimulus (Figure 1B), and it was observed with naked eye that the formed tooth-germ-like tissues were 2 times greater than the tissues formed in the control group (Figure 2). This is because mechanical stimulus promoted not only induction of differentiation of cells, but also promoted the growth of the cells, and also because the survival rate of the cells was increased. Furthermore, it was found that techniques of promoting the growth of tooth germ cells, including mechanical stimulus as a typical example, are applied to separate and culture a single tooth germ cell collected, so that multiple tooth germs can be regenerated from the single cell.

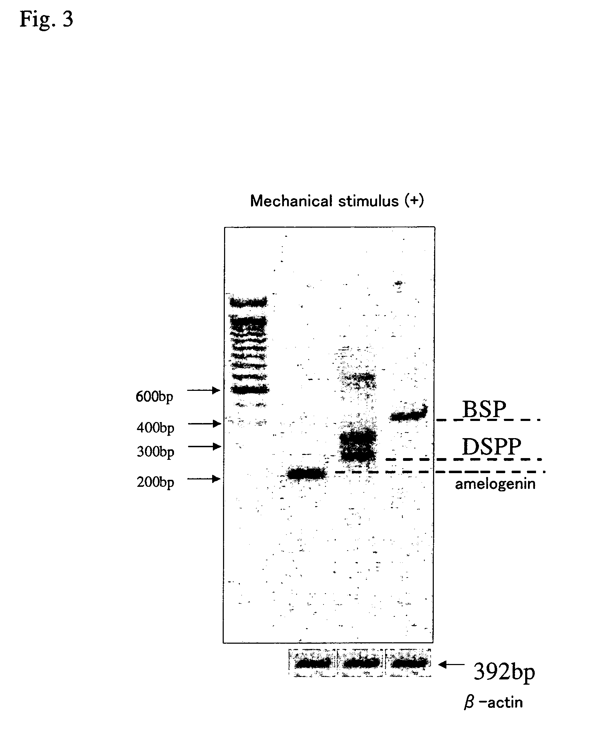

Tooth-germ-like tissues obtained at 15 weeks after transplantation were evaluated by RT-PCR. As a result, as shown in Figure 3, mRNAs of bone sialoprotein (BSP) and dentin sialophosphoprotein (DSPP), which are markers of odontoblasts that form dentin, and mRNA of amelogenin which is a marker of ameloblasts that form dental enamel, were expressed. Thus, it was found that tissues constituting tooth germ were regenerated.

### Example 2: Comparison of ability to form hard tissues in vitro

By the same method as described in Example 1, 1 × 10⁷ cells isolated from a fresh swine were inoculated on approximately 10 mg of a polyglycolic acid carrier (a carrier obtained by coating a polyglycolic acid mesh (manufactured by Albany International Research Co.) with type I collagen), so that the cells were allowed to adhere thereto. The carrier on which the cells were inoculated was placed in a medium. In an experiment group, the carrier was shaken at 50 times/minute in a shaker for 24 hours. In a control group, a static culture was carried out for 24 hours. Thereafter, for each group, the culture was continued for 7 days. As a medium for culturing the cells, a medium obtained by adding 10% fetal bovine serum, 2% glutamic acid, and 2% penicillin/streptomycin (100 units/100 µg/ml) to Minimum Essential Medium was used. The culture of the cells was carried out at 37°C in an atmosphere containing 5% carbon dioxide.

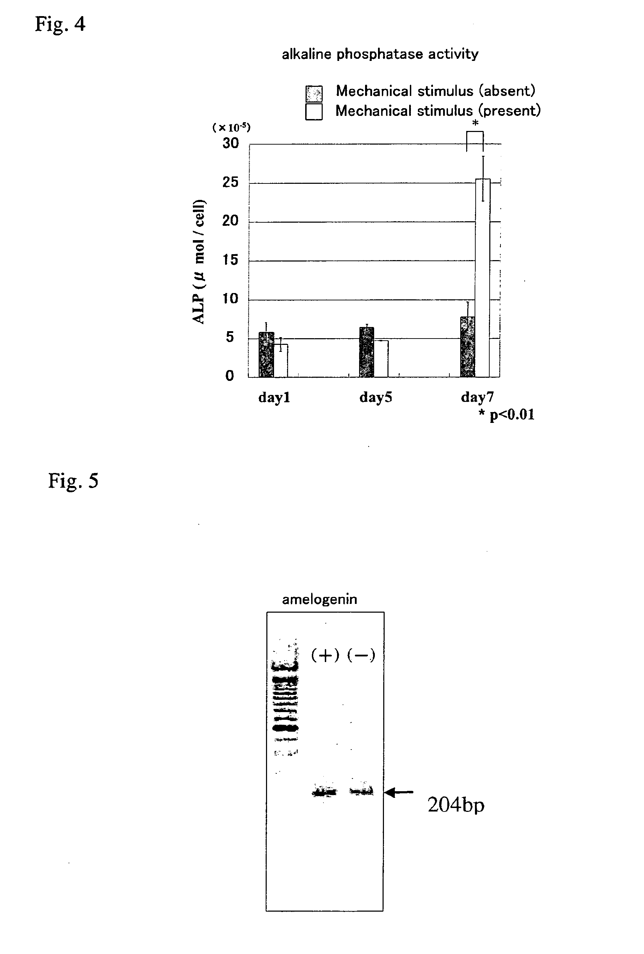

At 1st, 5th, and 7th days after culture, the number of cells was counted with a WST-8 kit, and alkaline phosphate activity was then measured (by Lowry's method). The results are shown in Figure 4.

The results shown in Figure 4 show that, in the cell group to which mechanical stimulus was given, alkaline phosphatase activity which is a marker of cells forming hard tissues significantly increased at 7th day after culture. Thus, it was found that mechanical stimulus gives advantageous effects on regeneration of tooth germ.

Moreover, using cells that were cultured for 7 days, the two cell groups were compared by RT-PCR in terms of the expression level of mRNA of amelogenin which is a marker of ameloblasts that form dental enamel. The results are shown in Figure 5.

The results shown in Figure 5 show that the cell group to which mechanical stimulus was given expressed a larger amount of amelogenin than that of the control group. Thus, it was found that mechanical stimulus gives advantageous effects on regeneration of tooth germ.

### INDUSTRIAL APPLICABILITY

Using the method of the present invention, a tooth germ structure such as dentin, dental papilla or enamel pulp is formed in the body of a transplanted animal or on a medium, or a cell mass is induced to differentiate such that it forms a tooth germ structure. Thereafter, such a tooth germ structure or cell mass is transplanted into the jaw of a dental patient together with a carrier, so as to regenerate a tooth root or tooth. As a result, this becomes a treatment that is extremely effective for recovery of the chewing ability of a patient who has lost a tooth. In addition, such a regenerated tooth enables esthetic recovery, and it greatly contributes to the improvement of the quality of life (QOL) of patients. Moreover, in the method of the present invention, physiologically active substances such as a growth factor or transcription factor may be used, so that a period of time required for regeneration of a tooth can be reduced, or that induction of differentiation of cells can be promoted.

## Claims

1. A method for regenerating tooth germ by culturing at least one type of tooth germ cells and any cells capable of differentiating into the tooth germ cells, while giving mechanical stimulus to the cells.

2. The method for regenerating tooth germ according to claim 1 wherein said at least one type of cells are odontoblasts, ameloblasts, pulp or dental papilla cells, tooth sac cells, or precursor cells thereof.

3. The method for regenerating tooth germ according to claim 1 or 2 wherein said at least one type of cells are obtained by fragmenting tissues collected from a living body, treating the fragmented tissues with enzyme, and separating and recovering them.

4. The method for regenerating tooth germ according to any of claims 1 to 3 wherein said at least one type of cells are inoculated on a carrier, and the cells are cultured on the carrier, while giving mechanical stimulus thereto.

5. The method for regenerating tooth germ according to claim 4 wherein as said carrier, there is used a carrier, the material of which has affinity to a living body into which the cultured cells are transplanted and an ability to be absorbed into the living body, and which has a form of interest to be regenerated and also has a portion into which blood circulation is introduced.

6. The method for regenerating tooth germ according to claim 4 wherein said carrier consists of at least one selected from polyglycolic acid (PGA), poly(DL-lactide-co-glycolide) (PLGA), poly(DL-lactide) (PLLA), caprolactone, collagen, or natural materials such as dentin.

7. The method for regenerating tooth germ according to any of claims 4 to 6 wherein said carrier has at least one form selected from mesh, sponge, or gel.

8. The method for regenerating tooth germ according to any of claims 1 to 7 wherein, as said mechanical stimulus, at least one mechanical stimulus selected from shake culture, ultrasonification, extension stimulus, or culture under pressure, is given to said at least one type of cells.

9. The method for regenerating tooth germ according to any of claims 1 to 8 wherein said at least one type of cells, which have been cultured while giving said mechanical stimulus thereto, are transplanted into the body of an animal, so as to allow tooth germ to regenerate in the body of said animal.

10. The method for regenerating tooth germ according to claim 9 wherein said animal is a mammal.

11. The method for regenerating tooth germ according to claim 9 or 10 wherein said at least one type of cells are transplanted into a site of the body of said animal, which has a high blood flow.

12. The method for regenerating tooth germ according to claim 8 wherein said at least one type of cells are inoculated on said carrier, and the cells are subjected to shake culture on the carrier at a frequency of approximately 50 times/minute, so as to promote the induction of differentiation and the growth.

13. The method for regenerating tooth germ according to claim 9 which comprises inoculating said at least one type of cells on said carrier, subjecting the cells to shake culture on the carrier at a frequency of approximately 50 times/minute, so as to promote the induction of differentiation and the growth, transplanting the cells into the body of an animal, and excising the regenerated tooth germ after approximately 15 weeks.

14. A tooth germ regenerated by the method of any of claims 1 to 13.

15. The regenerated tooth germ according to claim 14 which comprises at least one selected from dentin, dental papilla, or enamel pulp.

16. A treatment method wherein tooth germ regenerated by the method of any of claims 1 to 13 is transplanted into the jaw of a patient who has lost his or her own tooth germ or has had his or her own tooth germ damaged, so as to provide the patient with the regenerated germ tooth.
